# EUROPÄISCHE PATENTSCHRIFT

(11) **EP 0 761 670 B1**
(45) Veröffentlichungstag und Bekanntmachung des Hinweises auf die Patenterteilung: **13.02.2002**
(21) Anmeldenummer: 96113237.0
(22) Anmeldetag: 19.08.1996
(51) Int. Cl.: C07D 498/04, C08G 18/78

(54) **Polycyclische Iminooxadiazindione, deren Herstellung und Verwendung**
Polycyclic Iminoxadiazindiones, their preparation and use
Iminoxadiazindiones polycycliques, leur préparation et utilisation

(30) Priorität: 31.08.1995 DE 19532060
(43) Veröffentlichungstag der Anmeldung: 12.03.1997
(73) Patentinhaber: BAYER AG, 51368 Leverkusen (DE)
(72) Erfinder: Richter, Frank, Dr., 51373 Leverkusen (DE); Halpaap, Reinhard, dr., 51519 Odenthal (DE); Engbert, Theodor, Dr., 50968 Köln (DE)

(56) Entgegenhaltungen:
- EP-A- 0 081 712
- EP-A- 0 379 914

## Beschreibung

Die Erfindung betrifft neue polycyclische Iminooxadiazindione, ein Verfahren zu deren Herstellung und deren Verwendung.

Die Umsetzung von Isocyanaten mit H-aciden Verbindungen zur Herstellung hochmolekularer Stoffe ist seit langem bekannt (z.B. DRP 728 981) und hat eine breite industrielle Anwendung gefunden. Auf dem Lacksektor können niedermolekulare Diisocyanate aufgrund ihres hohen Dampfdruckes jedoch nur begrenzt verwendet werden. Deshalb bedient man sich verschiedener Verfahren zur Modifizierung dieser Monomeren mit dem Ziel, den Dampfdruck der resultierenden Produkte wesentlich zu erniedrigen. Beispiele hierfür sind die teilweise Umsetzung von Diisocyanaten mit zwei- und höherwertigen Alkoholen (Prepolymerisierung), die Herstellung von Polyisocyanaten mit Uretdion-("Dimer")-, Isocyanurat-("Trimer")- sowie Biuretstrukturelementen.

Die hohe Reaktivität der NCO-Gruppen von Isocyanaten muß für viele Anwendungen auf dem Lacksektor (z.B. 1K-Anwendungen mit möglichst langer Topfzeit, Polyurethan-Pulverlacksysteme, wäßrige Systeme usw.) herabgesetzt werden, z.B. durch eine thermisch oder chemisch reversible Blockierung der NCO-Gruppen mit Blockierungsmitteln, die bei der Vernetzungsreaktion zum polymeren Kunststoff oder Lackfilm wieder, gegebenenfalls in veränderter Form, abgespalten werden (z.B. Progr. Org. Coatings, 3 (1975), 73 und 9 (1981), 3). So können NCO-Gruppen z.B. mit ε-Caprolactam, Malonsäuredialkylester, Butanonoxim usw. reversibel blockiert werden.

Ein Nachteil bei der Verwendung blockierter Isocyanate ist, daß die bei der Polymerisation abgespaltenen Blockierungsmittel bei der anschließenden Aushärtung entweder entfernt werden müssen oder, sofern sie im Kunststoff oder der Beschichtung verbleiben, deren Eigenschaftsprofil nachteilig beeinflussen (z.B. Ausschwitzen, Verschlechterung der physikalischen und/oder chemischen Beständigkeit der Produkte).

Lackpolyisocyanate auf Uretdionbasis ("Dimerisate") vermeiden diese Nachteile, da sie sich thermisch induziert in die (Ausgangs)isocyanate rückspalten lassen.

Uretdione weisen jedoch eine Reihe von anderen Nachteilen auf. Einerseits kann bei längerer Lagerung, insbesondere bei höherer Temperatur, eine langsame Aufspaltung der Uretdionringe einsetzen, die zu einer Erhöhung des Restmonomerengehaltes in den Produkten führt. Andererseits erfolgt die zur schnellen Aushärtung des Kunststoffes bzw. der Beschichtung notwendige thermisch induzierte Spaltung der Uretdionringe erst bei relativ hohen Temperaturen, was zu Verfärbungen und anderen unerwünschten Zersetzungserscheinungen führen kann.

Aus der EP-A 14 365 sind den Uretdionen vergleichbare sogenannte selbstblockierte Verbindungen vom α-Nylon-Typ bekannt. Allerdings setzt die zur Vernetzung nötige Rückspaltung dieser Polymerisate bei noch höheren Temperaturen ein, als das bei den Uretdionen der Fall ist, weshalb ihr Einsatz, insbesondere für Lackanwendungen, bisher nicht erfolgte. Hinzu kommt, daß die thermisch induzierte Rückspaltung der α-Nylonverbindungen unter Freisetzung monomerer Diisocyanate verläuft.

Aufgabe der vorliegenden Erfindung war es daher, ein vernetzungsfähiges System zu entwickeln, in dem kein, oder zumindest ein im Vergleich mit den herkömmlichen, Blockierungsmittel-haltigen Systemen des Standes der Technik deutlich verringerter Anteil an Blockierungsmitteln enthalten ist und bei dem, auch bei höherer Temperatur, keine Rückspaltung in monomere Diisocyanate zu erwarten ist. Gleichzeitig sollte, ähnlich den Uretdiongruppen enthaltenen Systemen, ein möglichst hoher Anteil der im Ausgangs(di)isocyanat vorhandenen NCO-Gruppen für die Vernetzung zur Verfügung stehen.

Gegenstand der vorliegenden Erfindung sind polycyclische Iminooxadiazindione der Formel (I) 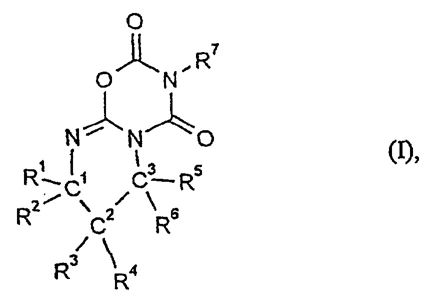 in welcher
- R¹ bis R⁶: unabhängig voneinander für H, C₁-C₁₀-Alkyl, C₆-C₂₄-Cycloalkyl, C₇-C₂₄-Aralkyl, C₇-C₂₄-Aryl stehen,
- R³ und R⁴: auch für gleiche oder verschiedene funktionelle Substituenten stehen können, ausgewählt aus der Gruppe Halogen, Oxo, C₁-C₁₀ Alk- oder C₆-C₂₄-Aryl-oxi, C₁-C₂₀-Alkoxycarbonyl, C₆-C₂₄-Aroyl,
wobei die Reste gegebenenfalls mit NCO-Gruppen, Halogen, wie Cl, Br, C₁-C₁₀-Alk- oder C₆-C₂₄-Aryl-oxi, C₁-C₁₀-Alkoxycarbonyl, C₆-C₂₄-Aroyl-Gruppen substituiert sein können und weiterhin
- R¹ und R⁶: zusammen mit den C¹-C³ Kohlenstoffatomen des Iminooxadiazindionringsystems einen weiteren, gegebenenfalls Heteroatome (O, N, S) enthaltenden, Ring mit mindestens 4 C-Atomen bilden, und
- R⁷: für einen Rest der Formel (II) 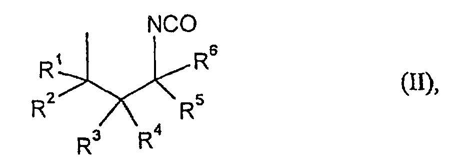
steht,
in dem
- R¹ bis R⁶: die bei Formel (I) genannten Bedeutungen haben können.

Gegenstand der Erfindung ist weiterhin ein Verfahren zur Herstellung der polycyclischen Iminooxadiazindione der Formel (I) 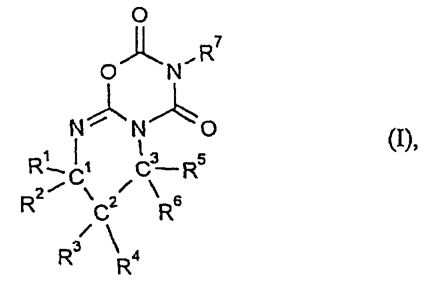 dadurch gekennzeichnet, daß Polyisocyanate der Formel (III) 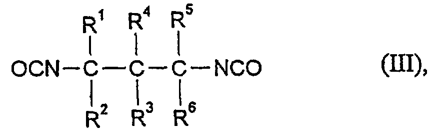 in welcher
- R¹ bis R⁶: die bei Formel (I) angegebene Bedeutung haben,
gegebenenfalls im Gemisch mit Mono- und/oder Polyisocyanaten, gegebenenfalls in einem Lösungsmittel in Anwesenheit eines Katalysators zu den erfindungsgemäßen Iminooxadiazidionen der Formel (I) umgesetzt werden.

Es können gegebenenfalls weitere, übliche Polyisocyanate zugemischt werden.

Die Erfindung betrifft ferner die Verwendung der erfindungsgemäßen polycyclischen Iminooxadiazindione der Formel (I) als Zwischenprodukt und als Komponente zur Herstellung von gegebenenfalls geschäumten Polyurethankunststoffen, Lacken und Beschichtungsmitteln.

Der Erfindung liegt die überraschende Beobachtung zugrunde, daß 1,3-Diisocyanatoalkane der Formel (III) gegebenenfalls in Gegenwart anderer Isocyanate, katalytisch induziert in die erfindungsgemäßen polycyclischen Iminooxadiazindione überführt werden können.

Dies ist deshalb überraschend, da in der Literatur an vielen Beispielen beschrieben wurde, daß (cyclo)aliphatische 1,3-Diisocyanate in einem weiten Temperaturbereich bei Anwesenheit verschiedener Katalysatoren ausschließlich zur (Cyclo)polymerisation unter Bildung hochmolekularer Verbindungen vom α-Nylon-Typ neigen (Chem. Commun. 4 (1966), 85; Organic Magnetic Resonance 2 (1970), 439; J. Makromol. Sci.-Chem A5(1) (1971) 37; "Organic Chemistry", A Series of Monographs, part 2, vol. 13 B, 2, S. 332 ff.; EP-A 14 365, 01.02.1979). Die Bildung von Iminooxadiazindionen wurde in keinem Falle beobachtet.

Slotta und Tschesche erhielten das monocyclische Trimethylderivat 3,5-Dimethyl-2-methylimino-4,6-diketo-1,3,5-oxadiazin, neben anderen Produkten, bei der Umsetzung von Methylisocyanat in Gegenwart von Tributylphosphin (Chem. Ber., 60 (1927), 295 und 1011). Diese Verbindung soll in besserer Ausbeute zugänglich sein, wenn in 1,2-Dichlorethan als Lösungsmittel gearbeitet wird (C.R. Acad. Sci. Ser. C 277 (1973) 795).

Das formal aus einem Äquivalent Phenyl- und zwei Äquivalenten Methylisocyanat aufgebaute monocyclische 3-Phenyl-5-methyl-2-methylimino-4,6-diketo-1,3,5-oxadiazin entsteht, neben anderen Produkten, bei der Reaktion von Diphenyl-methylimidocarbonat mit Tosylisocyanat (Chem. Ber. 120 (1987), 339).

In der DE-A 39 02 078 wird die Bildung des monocyclischen Tris(6-isocyanatohexyl)derivates (3,5-Bis(6-isocyanatohexyl)-2-(6-isocyanatohexyl)imino-4,6-diketo-1,3,5-oxadiazin) in untergeordnetem Maße bei der Trimerisierung von Hexamethylendiisocyanat in Gegenwart von Kohlendioxid erwähnt.

Die erfindungsgemäßen polycyclischen Iminooxadiazindione der Formel (I) lassen sich in einfacher Weise durch katalytisch induzierte Reaktion eines Poly-, bevorzugt Diisocyanates herstellen, in dem mindestens zwei Isocyanatgruppen als Substituenten an einem, gegebenenfalls substituierten, gegebenenfalls Heteroatome enthaltenden, gegebenenfalls cyclischen, aliphatischen System in 1,3-Stellung zueinander angeordnet sind, wobei gegebenenfalls zusätzlich auch andere Mono- oder Polyisocyanate als Reaktionspartner verwendet werden können.

Bevorzugt setzt man als (cyclo)aliphatische 1,3-Diisocyanate Verbindungen des Molekulargewichtsbereiches 126 bis 500, besonders bevorzugt 126 bis 350, ein. Beispiele für derartige 1,3-Diisocyanate sind 1,3-Propandiisocyanat, 1,3-Butandiisocyanat, 1,3-Pentandiisocyanat, 2,4-Pentandiisocyanat und andere Isomere der vorstehend genannten Diisocyanate mit 1,3-Stellung der Isocyanatgruppen zueinander wie z.B. 2,2-Dimethylpropan,-1,3-diisocyanat. Im Falle cycloaliphatischer Polyisocyanate weist der direkt mit den zur Bildung der erfindungsgemäßen Iminooxadiazindionstruktur in 1,3-Stellung verknüpfte Cyclus im allgemeinen 4 bis 8, vorzugsweise 5 oder 6, besonders bevorzugt 6 Kohlenstoffatome auf und kann beliebige inerte Substituenten, insbesondere Alkylgruppen, vorzugsweise solche mit 1 bis 4 Kohlenstoffatomen, aufweisen.

Besonders bevorzugt sind solche 1,3-Diisocyanatocycloalkane, die in 2- und/oder 4-Position einen oder mehrere C₁-C₄-Alkylsubstituenten aufweisen. Geeignete 1,3-Diisocyanatocycloalkane sind beispielsweise 1,3-Diisocyanatocyclobutan, 1,3-Diisocyanatocyclopentan, 1,3-Diisocyanatocyclohexan, 1,3-Diisocyanatocyclooctan, 1,3-Diisocyanato-2-methylcyclohexan, 1,3-Diisocyanato-4-methylcyclohexan sowie beliebige Gemische der beiden letztgenannten Diisocyanate, 1,3-Diisocyanato-2-isopropylcyclohexan, 1,3-Diisocyanato-4-isopropylcyclohexan, 1,3-Diisocyanato-2,4-dimethylcyclohexan, 1,3-Diisocyanato-2,4-diethylcyclohexan, 1,3-Diisocyanato-2,4-diethyl-6-methylcyclohexan, 1,3-Diisocyanato-2-methyl-4,6-diethylcyclohexan sowie beliebige technische Gemische der beiden letztgenannten Diisocyanate oder 1,3-Diisocyanato-2,4,6-triisopropylcyclohexan sowie 1,3-Diisocyanato-2,4,6-tributylcyclohexan.

Bei diesen 1,3-Diisocyanaten handelt es sich um bekannte Verbindungen, die z.B. durch Gasphasenphosgenierung der ihnen zugrundeliegenden Diamine hergestellt werden können. Diese Gasphasenphosgenierung ist z.B. in der DE-A 44 12 327.2 beschrieben. Zum Beispiel wird 1,3-Diisocyanato-4-isopropylcyclohexan (Stereoisomerengemisch) aus 1,3-Diamino-4-isopropylcyclohexan (Isomerengemisch) durch Gasphasenphosgenierung gewonnen und fällt in nahezu quantitativer Ausbeute als farblose Flüssigkeit mit einem Siedebereich von 120 bis 126 bei einem Druck von 0,1 mbar an.

Ganz allgemein fallen die durch Phosgenierung der entsprechenden cycloaliphatischen 1,3-Diamine erhaltenen Diisocyanate häufig als cis-trans-Stereoisomerengemische, bezogen auf die Stellung der beiden Isocyanatgruppen zueinander, mit einem cis-trans-Verhältnis von 5:1 bis 1:2, vorzugsweise 3:1 bis 1:1, an und sind als solche bereits prinzipiell zur Darstellung der erfindungsgemäßen polycyclischen Iminooxadiazindione geeignet.

Da bei letzteren, insbesondere bei Verwendung von Isomerengemischen mit einem hohen trans-Anteil, diese Isomeren auch anderen Modifizierungsreaktionen wie z.B. der Trimerisierung unter Ausbildung von Isocyanuratstrukturen, der Carbodiimidisierung sowie der Dimerisierung unter Ausbildung von Uretdionstrukturen zugänglich sind, können die erfindungsgemäßen polycyclischen Iminooxadiazindione der Formel (I) auch im Gemisch mit den genannten und weiteren Isocyanat-Folgeprodukten anfallen. Weiterhin können sie neben (Cyclo)polymerisaten (α-Nylonverbindungen) anfallen, von denen sie gegebenenfalls, beispielsweise durch Extraktion, abgetrennt werden können.

Die erfindungsgemäßen polycyclischen Iminooxadiazindione können auch in reiner Form erhalten werden, indem man von 1,3-Diisocyanatoalkanen oder, im Fall der cycloaliphatischen Vertreter, von reinen 1,3-cis-Diisocyanatocycloalkanen ausgeht. Letztere können, beispielsweise durch thermisch induzierte Depolymerisation von cyclopolymeren α-Nylonverbindungen, z.B. nach der Lehre der EP-A 14 365, hergestellt werden.

Das erfindungsgemäße Verfahren kann in Gegenwart eines homogen- oder heterogen Katalysators durchgeführt werden. Für die Homogenkatalyse eignen sich prinzipiell alle in der Literatur zur Herstellung von Oligoisocyanaten mit Isocyanurat- oder Uretdionstruktur zitierten Verbindungen wie z.B. Trialkylphosphine, quaternäre Ammoniumsalze wie Trimethylbenzylammoniumhydroxid, Si-N-Verbindungen wie Hexamethyldisilazan u.a. (vgl. J. prakt. Chem. 336 (1994), 185).

Als trägerfixierte Katalysatoren (Heterogenkatalyse) eignen sich ebenfalls alle u.a. zur Uretdionbildung ("Dimerisierung") sowie zur Herstellung von Isocyanuraten aus Isocyanaten bekannten Verbindungen, z.B. adsoptiv an Trägermaterialien wie Al₂O₃ oder Kieselgel gebundene oder insbesondere an Polystyrol polymergebundene Verbindungen des o.g. Typs, die beispielsweise in der EP-A 0 447 516 oder WO 93/18014 vorbeschrieben sind.

Die Reaktion kann in Substanz, gegebenenfalls aber auch unter Verwendung eines Lösungsmittels und gegebenenfalls bei einem anderen als dem Normaldruck, durchgeführt werden. Wird in Verdünnung gearbeitet, ist die Verwendung von Lösungsmitteln, die bei dem angewandten Druck oberhalb 150°C sieden, bevorzugt. Die Reaktion kann gegebenenfalls unter Inertgas, z.B. Argon, Stickstoff etc., durchgeführt werden.

Die Reaktion kann nach einem beliebig gewählten Umsetzungsgrad der monomeren Verbindung(en), beispielsweise durch Zugabe eines Katalysatorgiftes, durch thermische Desaktivierung des Katalysators oder durch einfaches Abkühlen des Reaktionsgemisches abgestoppt werden. Im letzteren Fall ist bei der Katalysatorwahl darauf zu achten, daß der Katalysator beim Durchlaufen des für die α-Nylonbildung kritischen Temperaturbereiches keine ausreichende (Rest)aktivität mehr besitzt oder daß dieser Temperaturbereich hinreichend schnell durchfahren wird ("Abschrecken"), so daß gegebenenfalls vorhandene Anteile an nicht umgesetzten Monomeren nicht oder nur in einem, für die Applikation des jeweiligen Produktes, tolerierbaren Ausmaß zu cyclopolymerem α-Nylon abreagieren. Führt man die Reaktion bis zum im wesentlichen vollständigen Verbrauch der 1,3-Diisocyanatoalkane durch, ist mit derartigen Problemen nicht zu rechnen. Anderenfalls kann anschließend vom verbliebenen, nicht umgesetzten Monomeren nach einer Methode des Standes der Technik wie z.B. Destillation, Dünnschichtdestillation oder Extraktion abgetrennt werden.

Nach einer besonderen, gegebenenfalls kontinuierlich arbeitenden, Ausführungsform des Verfahrens kann die Reaktion in einem Rohrreaktor vorgenommen werden, wobei hierfür der Einsatz von trägerfixierten Katalysatoren vorteilhaft ist, so daß nach katalytisch induzierter Herstellung der erfindungsgemäßen polycyclischen Iminooxadiazindione keine Desaktivierung andernfalls zuzusetzender Homogenkatalysatoren erfolgen muß.

Die erfindungsgemäßen polycyclischen Iminooxadiazindione der Formel (I) können, gegebenenfalls im Gemisch mit anderen Isocyanatfolgeprodukten, durch die üblichen Verfahren des Standes der Technik isoliert werden, wie z.B. Dünnschichtdestillation, Extraktion, Kristallisation oder Molekulardestillation und fallen dabei als farblose oder schwach gefärbte Flüssigkeiten oder Feststoffe an. Letztere weisen, abhängig von den eingesetzten Isocyanat(gemisch)en, einen Schmelzbereich von ca. 30 bis 150°C auf.

Die erfindungsgemäßen polycyclischen Iminooxadiazindione sind äußerst wertvolle Rohstoffe, die auch für die Verwendung im Kunststoff- und Lacksektor geeignet sind.

Sie können rein oder in Verbindung mit anderen Isocyanatderivaten des Standes der Technik, wie Uretdion-, Biuret-, Allophanat-, Isocyanurat-, Urethan- sowie Carbodiimid-Polyisocyanaten, deren freie NCO-Gruppen gegebenenfalls mit typischen Blockierungsmitteln desaktiviert wurden, eingesetzt werden.

Der besondere Vorteil der erfindungsgemäßen polycyclischen Iminooxadiazindione der Formel (I) ist zum einen darin zu sehen, daß sie, selbst bei längerer thermischer Belastung, keine Rückspalttendenz in die zugrundeliegenden monomeren (Di)isocyanate aufweisen, jedoch andererseits eine ausreichend hohe Reaktivität gegenüber Verbindungen, die Zerewitinoff-aktiven Wasserstoff enthalten, besitzen. Diese Beobachtungen sind insofern überraschend, als von den isomeren Isocyanuraten bekannt ist, daß dort das heterocyclische Ringsystem äußerst reaktionsträge ist. In der Literatur findet sich weiterhin nur ein Hinweis darauf, daß der Iminooxadiazindionring einer Solvolysereaktion unterworfen werden kann (Chem. Ber., 60 (1927), 295).

Tragen die Reste R¹ bis R⁷ noch andere reaktive Gruppen, beispielsweise Isocyanatgruppen, bieten sich die erfindungsgemäßen polycyclischen Iminooxadiazindione der Formel (I) bevorzugt für Anwendungen mit dualem Vernetzungsmechanismus an. Hierfür setzt man beispielsweise die freie(n) reaktiven Gruppen, z.B. Isocyanatgruppen, in einem ersten Reaktionsschritt mit einer Komponente Z, ähnlich der sogenannten "Vorverlängerung" von Di- bzw. Polyolen mit anderen, mindestens difunktionellen, Verbindungen um und führt in einem unabhängigen zweiten Schritt die Vernetzung unter Abbau der Iminooxadiazindionstruktur durch. Hierbei zeigt sich, daß bis zu zwei Reaktionspartner, die Zerewitinoff-aktiven Wasserstoff enthalten, je Äquivalent Iminooxadiazindioneinheit gebunden werden können. Durch die Anwesenheit weiterer reaktiver Gruppen im Molekül der erfindungsgemäßen polycyclischen Iminooxadiazindione der Formel (I) oder durch eine entsprechende Modifizierung im obengenannten "Vorverlängerungsschritt" bei Gegenwart einer, mehr als monofunktionellen, Komponente Z, läßt sich deshalb prinzipiell jede gewünschte Funktionalität einstellen.

Die resultierenden Kunststoffe und Beschichtungen entsprechen in ihrer chemischen Natur weitgehend denen, die auf Basis Biuret- sowie Allophanatgruppen enthaltender (Lack)rohstoffe (cyclo)aliphatischer Diisocyanate erhalten werden und sind somit außerordentlich hochwertige Produkte mit dem für die genannten, bewährten Systeme des Standes der Technik typischen Eigenschaftsprofil, ohne jedoch die bereits angesprochenen Nachteile aufzuweisen.

Die erfindungsgemäßen polycyclischen Iminooxadiazindione eignen sich für den Einsatz als Bindemittel in Beschichtungsmaterialien. Vorzugsweise werden sie, gegebenenfalls in Abmischung mit anderen typischen Rohstoffen, gegebenenfalls in "vorverlängerter" Form wie oben angeführt, als Vernetzerkomponente in, gegebenenfalls wäßrigen, 1- und 2-K-Beschichtungen eingesetzt. Bei der Verwendung als Vernetzerkomponente in 2K-Beschichtungen werden die erfindungsgemäßen Polyisocyanate in der Regel mit OH- sowie NH-Komponenten kombiniert, wie sie aus 2K-Polyurethansystemen an sich bekannt sind, so z.B. hydroxyfunktionellen Polyestern, Polyethern, Polycarbonaten, Polyurethanen, Polyacrylaten sowie polyfunktionellen Aminen.

Neben den erfindungsgemäßen Verfahrensprodukten und den gegebenenfalls mitverwendeten weiteren Bindemittelkomponenten und Lacklösungsmitteln oder Lacklösungsmittelgemischen wie beispielsweise Toluol, Xylol, Cyclohexan, Chlorbenzol, Butylacetat, Ethylacetat, Ethylglykolacetat, Methoxypropylacetat, Aceton, Testbenzin, höher substituierte Aromaten (Solventnaphtha® , Solvesso® , Shellsol® , Isopar® , Nappar® , Diasol® ) können in den Beschichtungen auch weitere Hilfs- und Zusatzmittel verwendet werden, wie z.B. Benetzungsmittel, Verlaufsmittel, Hautverhinderungsmittel, Antischaummittel, Mattierungsmittel, viskositätsregulierende Stoffe, Pigmente, Farbstoffe, UV-Absorber und Stabilisatoren gegen thermische und oxidative Einflüsse.

Die Beschichtungsmittel auf Basis der erfindungsgemäßen polycyclischen Iminooxadiazindione können zur Beschichtung einer Vielzahl von Materialien dienen, wie z.B. Holz, Kunststoff, Leder, Metall, Papier, Beton, Mauerwerk, Keramik und Textil.

### Beispiele

Alle Prozentangaben beziehen sich, wenn nicht anders angegeben, auf das Gewicht.

### Beispiel 1

200 g Hexahydro-toluylendiisocyanat ('H₆TDI': ein technisches Gemisch der regio- und stereoisomeren 1,3-Diisocyanato-2-(bzw. 4-)methyl-cyclohexane im ungefähren 2/4 Regioisomerenverhältnis 1:4, enthaltend ca. 60 % 1,3-cis-konfigurierte Stereoisomere) werden bei Zimmertemperatur in einem 1 l Vierhalskolben, versehen mit mechanischem Rührer, Innenthermometer, Rückflußkühler und Dosiereinrichtung für den Katalysator, vorgelegt, bei einem Druck <1 mbar entgast und anschließend bei 60°C mit 0,25 g, entsprechend 125 ppm, einer 10 %igen ®Triton B-Lösung in 2-Ethyl-1,3-hexandiol tropfenweise versetzt. Nach 2-stündigem Rühren bei dieser Temperatur wird auf 75°C erwärmt und nochmals dieselbe Menge Katalysatorlösung zugegeben. Durch Entfernen der Heizquelle und gegebenenfalls externes Kühlen mit einem Wasserbad wird die Innentemperatur der Mischung unter 100°C gehalten. Der titrimetrisch bestimmte NCO-Gehalt der Mischung ist nach Nachrühren von anfangs 46,6 % auf 30,3 % abgefallen. Anschließend wird durch Zugabe von 70 mg Dibutylphosphat die Reaktion unterbrochen ("abgestoppt") und die abgekühlte Mischung unter kräftigem Rühren in 800 ml getrocknetes n-Hexan eingebracht. Nach Abfiltrieren des ausgeschiedenen weißen Feststoffes (H₆TDI-α-Nylon) wird das Filtrat destillativ vom Lösungsmittel und nicht umgesetzten Monomeren befreit und der erhaltene farblose, bei Zimmertemperatur feste, Rückstand analysiert:
Ausbeute: 7,1 g;
titierter 'NCO'-Gehalt: 21,1 % wenn gemäß DIN 53 185 bei Zimmertemperatur gegen Dibutylamin titriert wird; 13,4 % wenn bei 0°C titriert wird;
gelpermeationschromatographisch bestimmter Gehalt an tricyclischem H₆TDI-Iminooxadiazindion (Isomerengemisch): 85 %.

Die Identität der Verbindung folgt zweifelsfrei aus den Resultaten folgender analytischer Untersuchungen: GPC, IR (auch als Kopplungsmethode präp. GPC-IR), ¹³C-NMR, Massenspektroskopie (auch als Kopplungsmethode GC-MS) und Elementaranalyse.

### Beispiel 2

Es wird wie unter Beispiel 1 verfahren mit der Ausnahme, daß als Katalysator 0,4 g, entsprechend 100 ppm, einer 5 %igen methanolischen Methylcholinlösung verwendet werden und die Reaktion bei einem titimetrisch bestimmten NCO-Gehalt der Mischung von 32,1 % abgestoppt wird. Ausbeute nach Aufarbeitung wie oben beschrieben: 9,4 g, 'NCO'-Gehalt (DIN 53 185) 21,1 %, gelpermeationschromatographisch bestimmter Gehalt an tricyclischem H₆TDI-Iminooxadiazindion (Isomerengemisch): 72 %.

### Beispiel 3

520 g 1,3-Diisocyanato-4-isopropylcyclohexan ('H₆CDI', technisches Gemisch von vier Stereoisomeren, enthaltend ca. 50 % 1,3-cis-konfigurierte Stereoisomere) wird wie im Beispiel 1 aufgeführt mit 6,4 g, entsprechend 60 ppm, einer 0,5 %igen ® Triton B-Lösung bis zu einem titrierten NCO-Gehalt der Mischung von 30,4 % (Ausgangswert: 40,4 %) umgesetzt, anschließend abgestoppt und aufgearbeitet wie beschrieben. Man erhält 28,3 g eines nahezu farblosen; bei Zimmertemperatur festen Rückstandes mit einem gelpermeationschromatographisch bestimmten Gehalt an tricyclischem H₆CDI-Iminoocadiazindion (Isomerengemisch) von 68 %.

### Beispiel 4

100 g 1,3-Diisocyanato-2,4-diethyl-6-methylcyclohexan ('H₆DETDI', technisches Gemisch mehrerer Stereoisomerer, enthaltend >30 % 1,3-cis-konfigurierte Stereoisomere) wird wie in Beispiel 1 aufgeführt mit 0,7 g, entsprechend 350 ppm, einer 5 %igen ® Triton B-Lösung bis zu einem titrierten NCO-Gehalt der Mischung von 30,4 % (Ausgangswert: 40,4 %) umgesetzt, anschließend abgestoppt und aufgearbeitet wie beschrieben. Man erhält 8,2 g eines farblosen, bei Zimmertemperatur festen Rückstandes mit einem gelpermeationschromatographisch bestimmten Gehalt an tricyclischem H₆CDI-Iminooxadiazindiol (Isomerengemisch) von 68 %.

### Beispiel 5

100 g 'H₆TDI' wie in Beispiel 1 werden nach dem Entgasen kurzzeitig in einem Ölbad auf 260°C erhitzt, mit 160 mg, entsprechend 640 ppm, einer 40 %igen ® Triton B-Lösung in Methanol versetzt. Nach fünfzehnminütigem Rühren bei dieser Temperatur wird durch Zugabe von 80 mg Dibutylphosphat die Reaktion unterbrochen ("abgestoppt") und die Mischung abgekühlt. Der NCO-Gehalt der erhaltenen Mischung ist, obwohl sie noch ca. 50 % monomeres "H₆TDI' enthält, stabil. Nach destillativer Entfernung des letzteren erhält man 27 g eines festen Rückstandes, der ca. 30 % tricyclisches H₆TDI-Iminooxadiazindion (Isömerengemisch) neben anderen H₆TDI-Folgeprodukten, hauptsächlich Isocyanurat-Polyisocyanaten, enthält. Der nach DIN 53 185 ermittelte NCO-Gehalt des Festharzes beträgt 23,9 %.

Das Gemisch ist in allen üblichen Lacklösungsmitteln wie z.B. Butylacetat, Methoxypropylacetat/Xylol-Gemisch (1:1) und ®Solvesso 100 gut löslich. Die Lösungen zeigen eine gute Verträglichkeit gegenüber den am Markt etablierten Lackpolyolen.

### Beispiel 6 (Anwendungsbeispiel)

10 g eines Polyisocyanatgemisches, erhalten nach der in Beispiel 5 beschriebenen Arbeitsweise, werden mit 12,3 g eines OH-funktionellen Polyesters auf Basis Phthalsäureanhydrid/Trimethylolpropan der OH-Zahl 260 (Äquivalentgewicht: 215 g/val_{OH}) in 15 g n-Butylacetat vermischt (NCO:OH-Verhältnis = 1:1), in einer 180 µm dicken Schicht auf eine Glasplatte aufgetragen und einer forcierten Trocknung für 30 Minuten bei 80°C unterworfen. Man erhält einen harten, klaren Film, der nach 80 Doppelhüben mit Aceton nur schwach angelöst wird.

Verwendet man einen 10 %igen Überschuß an OH-funktioneller Polyesterkomponente, was bei der Verwendung klassischer Isocyanathärter in einer deutlichen Verschlechterung der Lackeigenschaften des erhaltenen Filmes resultieren würde, und führt die Vernetzung für 60 Minuten bei 120°C durch, erhält man einen klaren Lackfilm, der auch nach 150 Doppelhüben mit Aceton keine Anzeichen einer Anlösung zeigt. Dieser Umstand kann der besseren Vernetzung zugeschrieben werden, die infolge Ringöffnung der Iminooxadiazindionringe bei höherer Temperatur einsetzt.

In Modellversuchen mit Dibutylamin bzw. Methanol konnte gezeigt werden, daß die Iminooxadiazindioneinheit bis zu 2 Äquivalente an NH- bzw. OH-funktioneller Komponente zu binden vermag.

### Beispiel 7 (Anwendungsbeispiel)

Ein Polyisocyanatgemisch, erhalten nach der in Beispiel I beschriebenen Arbeitsweise, mit einem Restmonomergehalt an "H₆TDI' von ≤ 0,03 % wird 2 Stunden lang bei 180°C gelagert. Der anschließend bestimmte Restmonomergehalt ist auf 0,04 % angestiegen. Dieser Versuch belegt, daß das 'H₆TDI'-Dimer mit tricyclischer Iminooxadiazindionstruktur (ca. 85 % des Gemisches, s.o.) keine Tendenz zur Rückspaltung in die zugrundeliegenden Ausgangsisocyanate aufweist. Mithin sind die erfindungsgemäßen Iminooxadiazindione im Gegensatz zu Verbindungen mit Uretdionstruktur, wie sie beispielsweise vom Hexamethylendiisocyanat sowie vom Isophorondiisocyanat bekannt sind und bei denen die quantitative Erfassung des Uretdiongehaltes häufig durch 'Heißtitration' erfolgt, da von einer vollständigen Rückspaltung der Uretdionringe bei 180°C ausgegangen werden kann (vgl. DE 37 39 549), äußerst rückspaltstabil.

## Patentansprüche

1. Polycyclische Iminooxadiazindione der Formel (I) 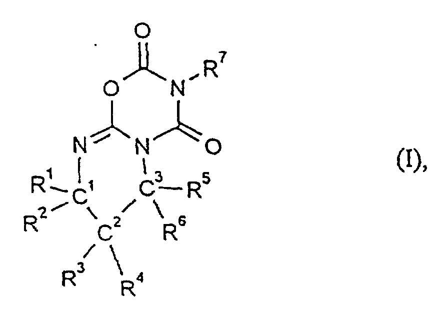 in welcher
R¹ bis R⁶ unabhängig voneinander für H, C₁-C₁₀-Alkyl, C₆-C₂₄-Cycloalkyl, C₇-C₂₄-Aralkyl, C₇-C₂₄-Aryl stehen, und
R³ und R⁴ auch für gleiche oder verschiedene funktionelle Substituenten stehen ausgewählt aus der Gruppe Halogen, Oxo, C₁-C₁₀-Alk- oder C₆-C₂₄-Aryl-oxi, C₁-C₂₀-Alkoxycarbonyl, C₆-C₂₄-Aroyl,
wobei die Reste gegebenenfalls mit NCO-Gruppen, Halogen, wie Cl, Br, C₁-C₁₀-Alk- oder C₆-C₂₄-Aryl-oxi, C₁-C₁₀-Alkoxycarbohyl, C₆-C₂₄-Aroyl-Gruppen substituiert sein können und weiterhin
R¹ und R⁶ zusammen mit den C₁-C₃ Kohlenstoffatomen des Iminooxadiazindionringsystems einen weiteren, gegebenenfalls Heteroatome (O, N, S) enthaltenden, Ring mit mindestens 4 C-Atomen bilden, und
R⁷ für einen Rest der Formel (II) 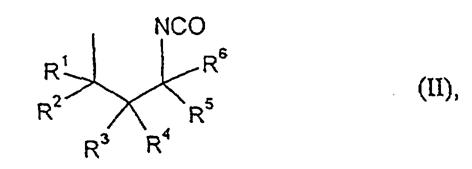
steht, in dem
R¹ bis R⁶ die bei Formel (I) genannten Bedeutungen haben können.

2. Verfahren zur Herstellung der polycyclischen Iminooxadiazindione der Formel (I) gemäss Anspruch 1 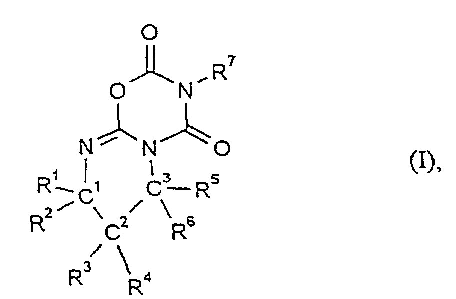 **dadurch gekennzeichnet, daß** Di- und Oligocyanate der Formel (III) 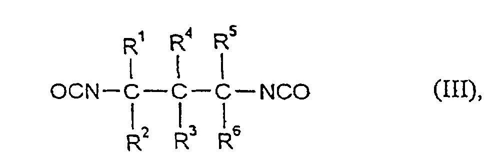 in welcher
R¹ bis R⁶ die bei Formel (I) angegebene Bedeutung haben,
gegebenenfalls im Gemisch mit Mono- und oder Polyisocyanaten, gegebenenfalls in einem Lösungsmittel in Anwesenheit eines Katalysators zu den erfindungsgemäßen polycyclischen Iminooxadiazindionen der Formel (I) umgesetzt werden.

3. Verfahren gemäß Anspruch 2, **dadurch gekennzeichnet, daß** die katalytisch induzierte Reaktion, gegebenenfalls in Gegenwart eines Lösungsmittels, bei Temperaturen zwischen -20°C und +600°Cdurchgeführt wird.

4. Verfahren gemäß Anspruch 2, **dadurch gekennzeichnet, daß** die katalytisch induzierte Reaktion, gegebenenfalls in Gegenwart eines Lösungsmittels, bei Temperaturen zwischen +20°C und +450°Cdurchgeführt wird.

5. Verfahren gemäß Anspruch 2, **dadurch gekennzeichnet, daß** die erfindungsgemäßen polycyclischen Iminaoxadiazindione durch Dimerisierung zweier Polyisocyanate hergestellt werden, in denen mindestens zwei Isocyanatgruppen als Substituenten an einem, gegebenenfalls substituierten, gegebenenfalls Heteroatome enthaltenden, alicyclischen Ringsystem in 1,3-Stellung zueinander angeordnet sind und daß in mindestens 20 % der Polyisocyanate diese Isocyanatgruppen cis-Konfiguration zueinander aufweisen.

6. Die Verwendung der Iminooxadiazindione gemäss Anspruch 1 als Komponente oder Zwischenprodukt zur Herstellung von, gegebenenfalls geschäumten, Polyurethankunststoffen, Lacken und Beschichtungsmitteln.

## Claims

1. Polycyclic iminooxadiazinediones of formula (I) 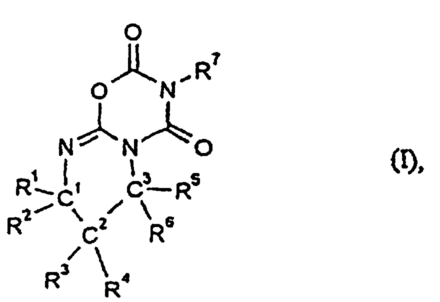 wherein
R¹ to R⁶ represent, independently of each other, H, C₁-C₁₀ alkyl, C₆-C₂₄ cycloalkyl, C₇-C₂₄ aralkyl, or C₇-C₂₄ aryl, and
R³ and R⁴ may also represent identical or different functional substituents selected from the group comprising a halogen, oxo, C₁-C₁₀ alkoxy or C₆-C₂₄ aryloxy, C₁-C₂₀ alkoxycarbonyl, or C₆-C₂₄ aroyl,
wherein the radicals may optionally be substituted with NCO groups, a halogen such as Cl or Br, C₁-C₁₀ alkoxy or C₆-C₂₄ aryloxy, C₁-C₁₀ alkoxycarbonyl, or C₆-C₂₄ aroyl groups, and in addition
R¹ and R⁶, together with the C₁-C₃ carbon atoms of the iminooxadiazinedione ring system, form a further ring containing at least 4 C atoms, which optionally contains heteroatoms (O, N, S), and
R⁷ represents a radical of formula (II) 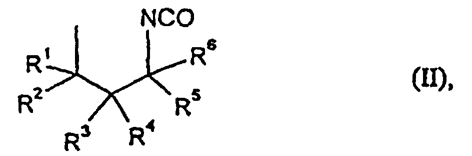
wherein
R¹ to R⁶ may have the meanings specified for formula (I).

2. A process for preparing polycyclic iminooxadiazinediones of formula (I) according to claim 1 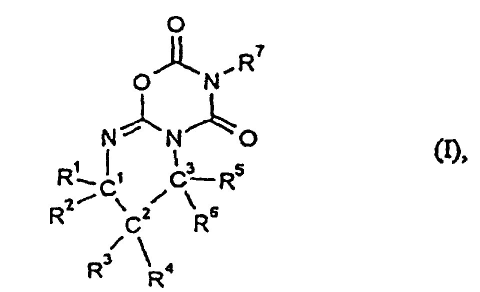 **characterised in that** di- and oligocyanates of formula (III) 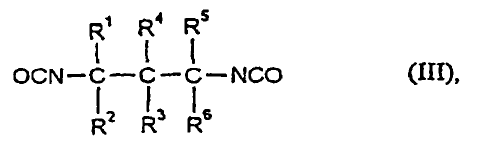 wherein
R¹ to R⁶ have the meanings specified for formula (I),
are converted in the presence of a catalyst to iminooxadiazinediones of formula (I) according to the invention, optionally in admixture with mono- and/or polyisocyanates, and optionally in a solvent.

3. A process according to claim 2, **characterised in that** the catalytically induced reaction is conducted at temperatures between -20° C and +600° C, optionally in the presence of a solvent.

4. A process according to claim 2, **characterised in that** the catalytically induced reaction is conducted at temperatures between +20°C and +450°C, optionally in the presence of a solvent.

5. A process according to claim 2, **characterised in that** the polycyclic iminooxadiazinediones according to the invention are prepared by the dimerisation of two polyisocyanates in which at least two isocyanate groups are disposed in the 1,3-position in relation to each other as substituents on an alicyclic ring system which is optionally substituted and which optionally contains heteroatoms, and that in at least 20 % of the polyisocyanates these isocyanate groups have a cis-configuration in relation to each other.

6. The use of the iminooxadiazinediones according to claim 1 as a component or intermediate product for the production of polyurethane plastics, which are optionally foamed, and for the production of lacquers and coating media.

## Revendications

1. Iminooxadiazinediones polycycliques de formule (I) 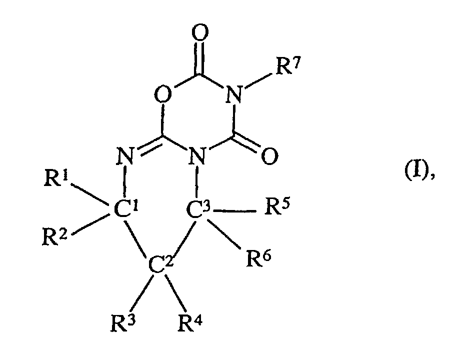 dans laquelle
R¹ à R⁶ représentent chacun, indépendamment les uns des autres, H, un groupe alkyle en C₁-C₁₀, cycloalkyle en C₆-C₂₄, aralkyle en C₇-C₂₄, aryle en C₇-C₂₄, et
R³ et R⁴ peuvent également représenter des substituants fonctionnels identiques ou différents choisis parmi les halogènes, les groupes oxo, alcoxy en C₁-C₁₀ ou aryloxy en C₆-C₂₄, (alcoxy en C₁-C₂₀)carbonyle, aroyle en C₆-C₂₄,
ces groupes pouvant, le cas échéant porter des substituants NCO, halogéno tel que Cl, Br, alcoxy en C₁-C₁₀ ou aryloxy en C₆-C₂₄, (alcoxy en C₁-C₁₀)carbonyle, aroyle en C₆-C₂₄, et
R¹ et R⁶ peuvent également former, avec les atomes de carbone C₁ à C₃ du système cyclique iminooxadiazinedione, un autre cycle à au moins quatre atomes de carbone contenant le cas échéant des hétéroatomes (O, N, S), et
R⁷ représente un groupe de formule (II) 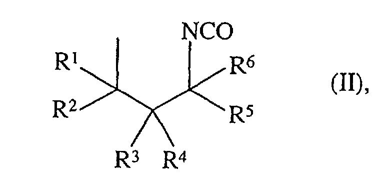
dans laquelle
R¹ à R⁶ ont les significations indiquées à la référence à la formule (I).

2. Procédé pour la préparation des iminooxadiazinediones polycycliques de formule (1) de la revendication 1, 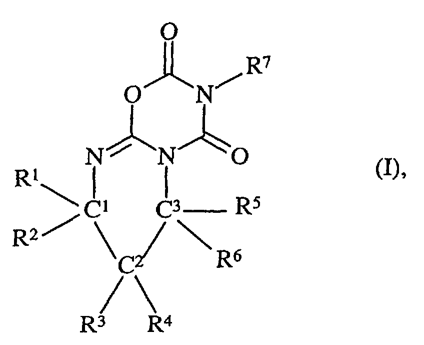 **caractérisé en ce que** l'on convertit des di- et oligo-cyanates de formule (III) 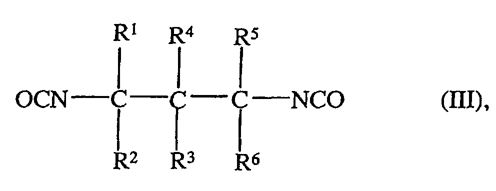 dans laquelle
R¹ à R⁶ ont les significations indiquées en référence à la formule (I), éventuellement en mélange avec des mono- et/ou des poly-isocyanates, éventuellement dans un solvant, en présence d'un catalyseur, en les iminooxadiazinediones polycycliques de formule (I) selon l'invention.

3. Procédé selon la revendication 2, **caractérisé en ce que** la réaction catalytique est réalisée, éventuellement en présence d'un solvant, à des températures allant de -20 à +600°C.

4. Procédé selon la revendication 2, **caractérisé en ce que** la réaction catalytique est réalisée éventuellement en présence d'un solvant à des températures allant de +20 à +450°C.

5. Procédé selon la revendication 2, **caractérisé en ce que** l'on prépare les iminooxadiazinediones polycycliques selon l'invention par dimérisation de deux polyisocyanates dans lesquels au moins deux groupes isocyanates, substituants d'un système alicyclique éventuellement substitué, contenant éventuellement des hétéroatomes, sont en position mutuelle 1,3 et, dans au moins 20 % des polyisocyanates, ces groupes isocyanates sont en configuration mutuelle cis.

6. L'utilisation des iminooxadiazinediones selon la revendication 1 en tant que composants ou produits intermédiaires de la préparation de résines synthétiques de polyuréthanne éventuellement gonflées en mousse, de vernis et de produits de revêtement.
